# EUROPEAN PATENT SPECIFICATION

(11) **EP 2 258 423 B1**
(45) Date of publication and mention of the grant of the patent: **17.08.2011**
(21) Application number: 09425223.6
(22) Date of filing: 04.06.2009
(51) Int. Cl.: A61M 5/24, A61M 5/315, A61M 5/32, A61M 5/34

(54) **Reusable syringe with replaceable tubular vial and retractable needle**
Mehrwegspritze mit austauschbarer Ampulle und zurückziehbarer Nadel
Seringue réutilisable avec ampoule et aiguille rétractable

(43) Date of publication of application: 08.12.2010
(73) Proprietor: Mariani, Umberto, 24042 Capriate S. Gervasio (BG) (IT)
(72) Inventor: Mariani, Umberto, 24042 Capriate S. Gervasio (BG) (IT)
(74) Representative: Gatti, Enrico

(56) References cited:
- EP-A- 0 337 252
- EP-A- 0 351 541
- FR-A- 2 680 111
- US-A- 4 808 169
- US-A- 5 382 235
- US-A- 5 403 288
- US-A1- 2003 073 958
- US-E1- R E37 908

## Description

The present invention relates to a tube vial for a multi-use syringe, a needle for use in combination with said tube vial, and a multi-use syringe for dental use.

The dental sector uses multi-use syringes composed of a metal body open on both sides, with a handgrip and a plunger operable by a ring or loop.

For their use, a vial known as a tube vial, in the form of a small tube with a fixed plug at its front and an internally slidable plug at its rear, has to be inserted into the syringe body.

A needle must also be fixed to the syringe. The needle is partially incorporated centrally into a plastic element connected to it, which comprises a thread for fitting it to the syringe. The thread is screwed onto the syringe, with the rear part of the needle pressure-perforating the fixed plug of the tube vial. Normally, this operation is carried out with the needle point covered by a suitable cap.

The syringe is now ready for the injection, after which the needle can be detached only and exclusively by an operation carrying a high risk of danger to health, consisting of covering the needle with its cap and unscrewing it. At that moment the needle and tube vial are inflected, and potentially able to infect by mechanical traumas, with possible spreading of organic residues from one of the two ends of the needle. The plunger has then to be withdrawn and the tube vial removed.

Document EP 337 252 relates to a safety syringe having a disposable, prefilled medication carpule and a combination needle retaining hub and needle cannula, and to a slide lock which is connected to the carpule and movable from a needle retaining position, at which the cannula is anchored so as to project axially from the carpule for administering an injection, to a needle releasing position, at which the cannula may be retracted within and completely shielded by the carpule.

Document EP 351 541 relates to a combination needle cannula and cannula lock which is to be interfaced with a prefilled medication carpule at the interior of a hypodermic syringe so that the cannula can either be locked at an axially extended position for administering an injection or released from the axially extended position and retracted within and completely surrounded by said carpule, whereby the cannula may be safely discarded after use while avoiding an accidental needle stick and the possible spread of disease. Document US 5,403,288 relates to a needle and sleeve assembly useful for use in association with a dental syringe comprising a hollow barrel, a piston, and an anaesthetic ampule cavity therein, comprising: a hollow elongated sleeve having openings at each end thereof; and a hollow housing base which supports at one end thereof an injection needle, and at the other end thereof, an ampule penetrating needle, the exterior of the housing base being adapted to slidably engage with the hollow interior of the sleeve. Document US 2003/073958 relates to a disposable aspirating syringe for dental medicine having the ability to accept single use or disposable drug cartridges. The syringe offers a retractable needle and a break-away plunger to facilitate disposal and increase safety.

Document FR 2 680 111 relates to means for holding the needle in the rubber, produced using a system for screwing the distal part of this needle into the corresponding part of the rubber.

Documents US 4,808,169 and US 5,382,235 disclose syringes with needles provided with a lateral groove or hole in the needle itself, in order to reduce the residual amount of drug in the vial at the end of the injection.

An object of the present invention is to provide a tube vial for a multi-use syringe, a needle for use in combination with said tube vial, and an autoclavable metal multi-use syringe which eliminates the aforesaid problems.

Another object of the present invention is to provide such a system, by which the extreme danger of the operation of covering the needle with its cap is avoided.

These and further objects are attained, according to the present invention, by a multi-use syringe, according to claim 1.

Further characteristics of the invention are described in the dependent claims.

The characteristics and advantages of the present invention will be apparent from the ensuing detailed description of one embodiment thereof, illustrated by way of non-limiting example in the accompanying drawings, in which:

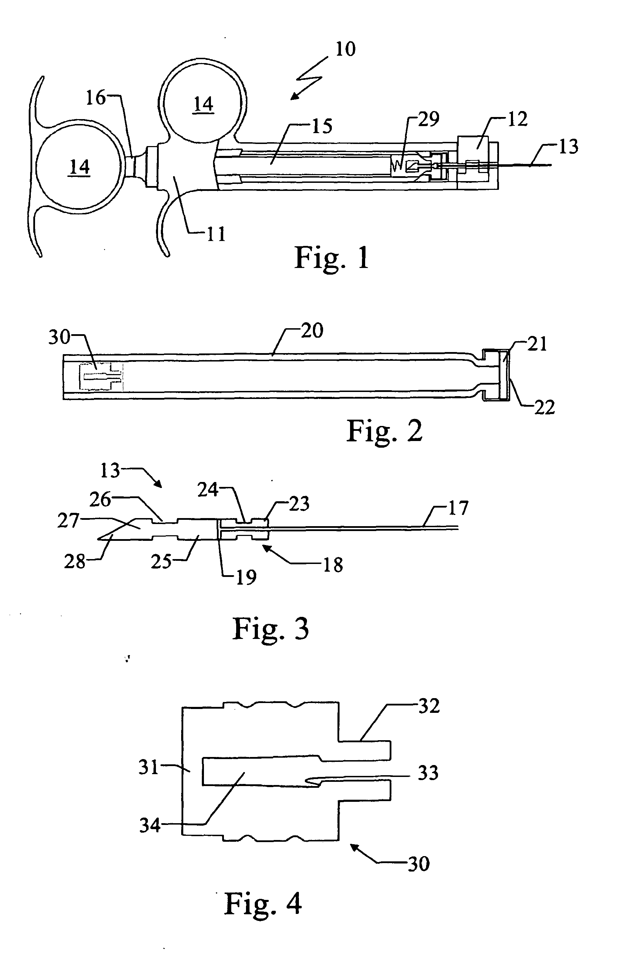

Figure 1 shows an autoclavable metal multi-use syringe in accordance with the present invention;
Figure 2 shows a tube vial in accordance with the present invention;
Figure 3 shows a needle in accordance with the present invention;
Figure 4 shows a detail of the tube vial plug in accordance with the present invention;

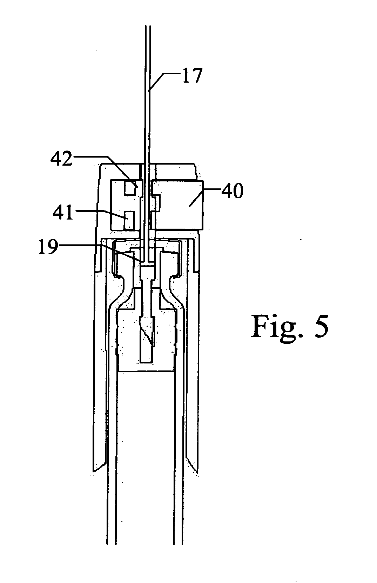

Figure 5 shows a final portion of a multi-use syringe in accordance with the present invention.

With reference to the accompanying figures, a multi-use syringe 10 is composed essentially of three parts.

A first part is the laterally open normally metal body 11 having at one end a system 12 for coupling to a needle 13, and at its other end suitable elements 14 for gripping the syringe 10. It comprises a plunger 15 connected to a ring or to suitable elements 16 for its operation.

A second part is the needle 13 comprising a hollow metal stem 17 which is partially embedded, at the end distant from the point of the needle 13, in a substantially cylindrical structure 18 of plastic or metal.

A third part is the tube vial 20, consisting of a transparent cylindrical body having its front end closed by a plug 21 of medical silicone material, fixed to the tube vial 20 by a normally metal plate 22. The plate 22 leaves the plug 21 free in its front central part, such that it can be perforated by the rear end of the needle 13. The rear end of the tube vial 20 is closed by a plug 30, preferably of medical silicone material, slidable within the cylindrical body.

The stem 17 is coaxially incorporated into the structure 18 for a predetermined distance.

At the end of the stem 17, in that portion thereof incorporated into the structure 18, this latter comprises a hole 19 passing transversely through the structure 18.

The hollow stem 17 terminates in the hole 19, such as to form a hydraulic T-joint between the hollow parts 17 and 19.

The structure 18 is preferably formed by co-moulding the stem 17, after which the hole 19 is made. The structure 18 can be previously formed of plastic or metal and then glued to the stem 17.

The dimensions (diameter) of the bore in the stem 17 and of the hole 19 are substantially equal and are of the order of the normal dimensions of a needle used in this field.

The structure 18 upstream of the hole 19 does not present any other hole and consists of solid plastic or metal.

The structure 18 must be formed of a sufficiently strong material as it has to be able to perforate the plug 21 of medical silicone material fixed to the tube vial 20.

The structure 18 has a substantially cylindrical shape with some diameter variations.

The connection of the structure 18 to the stem 17 commences with a portion 23 having a first diameter, then a portion 24 having a second diameter smaller than the first one. A further portion 25 having the first diameter. This first diameter then continues for most of the structure 18. There is then a portion 26 having a third diameter substantially equal to the second diameter. The final portion 27, having the first diameter, terminates with a central or lateral point 28 for perforating the plug 21.

The plug 30, slidable within the cylindrical body of the tube vial 20, comprises on its outer end a solid part 31 arranged to be engaged by the plunger 15. The plunger 15 is provided at its end with a sufficiently rigid metal wire 29 of corkscrew shape which by means of one or more revolutions of the end ring 16 of the plunger 15 penetrates into the solid part 31. Other systems for coupling the plunger 15 to the plug 30 can be used. For example, one or more wire pieces can be used which are inclined such that on rotating the plunger they penetrate into the solid part 31 of the plug.

At its inner end 32, and hence opposite the part 31, the plug 30 presents a diameter reduction such that when the plunger is completely thrust forwards, it can at least partially penetrate into the final portion of the tube vial 20, which also has a diameter reduction in its final part.

The plug 30 also presents a cavity 34 open towards the inside of the tube vial 20. The cavity 34 also has two different diameters; an initial first diameter of the cavity 34 and a final second diameter of the cavity 34, greater than the first diameter, such as to present at about the middle of the cavity a negative circular break-out 33.

The break-out 33 is necessary to retain the end 27 of needle 13 in its interior.

The system 12 for coupling to the needle 13 (needle lock) comprises a pressable block 40 counterbalanced by a spring 41 and having, in proximity to the axis of the syringe 10, at least one projection 42 for cooperating with the portion 24 of the structure 18.

The operation of the device of the invention is evident from that described and illustrated, and is essentially as follows.

The tube vial 20 is inserted into the syringe 10. The plunger 15 is inserted into the plug 30.

The needle 13 (including a needle cover, not shown) is inserted into the coupling system 12 after lightly pressing the needle block 40 so that the structure 18 can pass and travel against the plug 21 to perforate it, with the rear part 18 of the needle 13 passing through it.

The hole 19 then lies a few millimetres inside the tube vial 20.

The needle block 40 is then released so that the projection 42 fits into the portion 24 which is of smaller diameter than the main portion of the structure 18.

The syringe is now ready for use.

The injection is carried out by pressing the plunger 15, so that the liquid contained in the tube vial flows out.

After the injection, the plug 30 slidable within the tube vial 20 lies close to the rear of the needle 13. By further slightly forcing the plunger 15, the plug 30 further advances and the point 28 penetrates into the cavity 34. The edges of the structure 18 which are formed between the portions 26 and 27 abut against the break-out 33, so that the end of the needle 13 is retained by the plug 30.

By pulling the plunger 15 rearwards with one hand and pressing the block 40 of the release device 12 so that both hands are engaged, the needle 13 is led to the inside of the cylindrical body of the tube vial 20.

In this manner the needle 13 is isolated from the outside of the cylindrical body of the tube vial 20 and from the two plugs 21 and 30. The tube vial 20 with the needle 13 inside it can be removed from the syringe 10 and disposed of without problems.

The present invention provides important advantages.

It makes the use of a syringe, particularly of dental type, safe and immune from any possible infection which it can cause.

The transverse hole 19 enables all the liquid included in the tube vial to be used, in contrast to those needles having the hole at their rear end.

In practice the materials used and the dimensions can be chosen at will according to requirements and to the state of the art.

## Claims

1. A multi-use dental syringe (10) having at one end a system (12) for coupling to a needle (13), and at its other end suitable elements (14) for gripping the syringe (10); further comprising a plunger (15); said syringe comprising a tube vial inserted into said syringe, comprising a transparent cylindrical body (20) having a first end closed by a first plug (30) slidable within said cylindrical body (20) and a second end closed by a fixed second plug (21) perforable by the rear end of a needle (13); said plunger (15) comprising means for coupling it to said first plug (30); the syringe further comprising a needle (13) coupled to said system (12) wherein the z rear end of said needle (13) is incorporated into a structure (18) which extends beyond the rear end of said needle (13); **characterised in that** said structure (18) comprises a transverse hole (19) at the rear end of said needle (13) ,the hole (19) being in communication with the hollow stem (17) of said needle (13), and in communication with the inside of the tube vial (20) once the structure (18) perforates the second plug (21).

2. A syringe (10) as claimed in claim 1 **characterized in that** the system (12) for coupling to the needle (13) comprises a pressable block (40) counterbalanced by a spring (41) and having, in proximity to the axis of said syringe (10), at least one projection (42) for cooperating with a portion (24) of the structure (18).

3. A syringe (10) as claimed in claim 1, **characterized in that** said structure (18) comprises in succession a first portion (23) having a first diameter; a second portion (24) having a second diameter less than said first diameter; a third portion (25) having said first diameter; a fourth portion (26) having a third diameter substantially equal to the second diameter; a fifth portion (27) having said first diameter; and a point (28) arranged to perforate the plug (21) of the tube vial.

4. A syringe (10) as claimed in claim 1, **characterized in that** said plug (30) presents a cavity (34) open towards the inside of said tube vial (20); said cavity (34) having an initial first diameter and a final second diameter; said second diameter greater than said first diameter, such as to present a negative circular break-out (33), to retain the end (27) of said needle (13) in its interior, once the plug (30) has been fully advanced within the tube vial (20).

5. A syringe (10) as claimed in claim 1, **characterized in that** said transverse hole (19) forms a hydraulic T-joint together with hollow stem (17) of the needle (13) which terminates in hole (19).

6. A syringe (10) as claimed in claim 1, **characterized in that** said structure (18) is formed of plastic or metal.

## Patentansprüche

1. Mehrweg-Dentalspritze (10), die an einem Ende ein System (12) zur Kopplung mit einer Nadel (13) und an ihrem anderen Ende geeignete Elemente (14) zum Greifen der Spritze (10) aufweist; außerdem mit einem Kolben (15); wobei die Spritze eine rohrförmige Ampulle enthält, die in die Spritze eingesetzt ist und einen transparenten zylindrischen Körper (20) mit einem ersten Ende, das durch einen ersten Stöpsel (30) geschlossen ist, der in dem zylindrischen Körper (20) verschiebbar ist, und einem zweiten Ende aufweist, das durch einen feststehenden zweiten Stöpsel (21) geschlossen ist, der durch das hintere Ende einer Nadel (13) durchstochen werden kann; wobei der Kolben (15) Mittel aufweist, um ihn mit dem ersten Stöpsel (30) zu koppeln; wobei die Spritze außerdem eine Nadel (13) aufweist, die mit dem System (12) gekoppelt ist, wobei das hintere Ende der Nadel (13) in eine Struktur (18) integriert ist, die sich über das hintere Ende der Nadel (13) hinaus erstreckt; **dadurch gekennzeichnet, dass** die Struktur (18) ein schräg verlaufendes Loch (19) am hinteren Ende der Nadel (13) aufweist, wobei das Loch (19) mit dem hohlen Schaft (17) der Nadel (13) in Verbindung steht, und mit der Innenseite der rohrförmigen Ampulle (20) in Verbindung steht, wenn die Struktur (18) den zweiten Stöpsel (21) durchsticht.

2. Spritze (10) nach Anspruch 1, **dadurch gekennzeichnet, dass** das System (12) zur Kopplung mit der Nadel (13) einen pressbaren Block (40) aufweist, der durch eine Feder (41) ausgeglichen wird und in der Nähe der Achse der Spritze (10) mindestens einen Vorsprung (42) hat, um mit einem Bereich (24) der Struktur (18) zusammenzuwirken.

3. Spritze (10) nach Anspruch 1, **dadurch gekennzeichnet, dass** die Struktur (18) der Reihe nach einen ersten Bereich (23) mit einem ersten Durchmesser, einen zweiten Bereich (24) mit einem zweiten Durchmesser, der kleiner ist als der erste Durchmesser, einen dritten Bereich (25) mit dem ersten Durchmesser, einen vierten Bereich (26) mit einem dritten Durchmesser, der im Wesentlichen gleich dem zweiten Durchmesser ist, einen fünften Bereich (27) mit dem ersten Durchmesser, und eine Spitze (28) aufweist, die ausgestaltet ist, um den Stöpsel (21) der rohrförmigen Ampulle zu durchstechen.

4. Spritze (10) nach Anspruch 1, **dadurch gekennzeichnet, dass** der Stöpsel (30) einen Hohlraum (34) hat, der zur Innenseite der rohrförmigen Ampulle (20) offen ist; wobei der Hohlraum (34) einen ersten Anfangsdurchmesser und einen zweiten Enddurchmesser hat; wobei der zweite Durchmesser größer ist als der erste Durchmesser, um so einen negativen runden Durchbruch (33) zu bilden, um das Ende (27) der Nadel (13) in seinem Innerem zu halten, wenn der Stöpsel (30) vollständig die rohrförmige Ampulle (20) vorgeschoben ist.

5. Spritze (10) nach Anspruch 1, **dadurch gekennzeichnet, dass** das schräg verlaufende Loch (19) zusammen mit dem hohlen Schaft (17) der Nadel (13), der in dem Loch (19) endet, eine hydraulische T-Verbindung bildet.

6. Spritze (10) nach Anspruch 1, **dadurch gekennzeichnet, dass** die Struktur (18) aus Kunststoff oder aus Metall hergestellt ist.

## Revendications

1. Seringue dentaire polyvalente (10) ayant à une extrémité un système (12) pour se coupler à une aiguille (13), et à son autre extrémité des éléments adaptés (14) à la préhension de la seringue (10) ; comprenant en outre un piston (15) ; ladite seringue comprenant une ampoule insérée dans ladite seringue, comprenant un corps cylindrique transparent (20) ayant une première extrémité fermée par un premier bouchon (30) pouvant coulisser à l'intérieur dudit corps cylindrique (20) et une seconde extrémité fermée par un second bouchon fixe (21) perforable par l'extrémité arrière d'une aiguille (13) ; ledit piston (15) comprenant des moyens pour le coupler audit premier bouchon (30) ; la seringue comprenant en outre une aiguille (13) couplée audit système (12), l'extrémité arrière de ladite aiguille (13) étant incorporée dans une structure (18) qui s'étend au-delà de l'extrémité arrière de ladite aiguille (13) ; **caractérisée en ce que** ladite structure (18) comprend un trou transversal (19) à l'extrémité arrière de ladite aiguille (13), le trou (19) étant en communication avec la tige creuse (17) de ladite aiguille (13), et en communication avec l'intérieur de l'ampoule (20) une fois que la structure (18) perfore le second bouchon (21).

2. Seringue (10) selon la revendication 1, **caractérisée en ce que** le système (12) pour se coupler à l'aiguille (13) comprend un bloc enfonçable (40) équilibré par un ressort (41) et présentant, à proximité de l'axe de ladite seringue (10), au moins une saillie (42) pour coopérer avec une partie (24) de la structure (18).

3. Seringue (10) selon la revendication 1, **caractérisée en ce que** ladite structure (18) comprend successivement une première partie (23) ayant un premier diamètre, une deuxième partie (24) ayant un deuxième diamètre inférieur audit premier diamètre ; une troisième partie (25) ayant ledit premier diamètre ; une quatrième partie (26) ayant un troisième diamètre sensiblement égal au deuxième diamètre ; une cinquième partie (27) ayant ledit premier diamètre ; et un point (28) agencé pour perforer le bouchon (21) de l'ampoule.

4. Seringue (10) selon la revendication 1, **caractérisée en ce que** ledit bouchon (30) présente une cavité (34) ouverte vers l'intérieur de ladite ampoule (20) ; ladite cavité (34) ayant un premier diamètre initial et un second diamètre final ; ledit second diamètre étant supérieur audit premier diamètre, de sorte à présenter une rupture circulaire négative (33), pour retenir à l'intérieur l'extrémité (27) de ladite aiguille (13), une fois que le bouchon (30) a été complètement avancé à l'intérieur de l'ampoule (20).

5. Seringue (10) selon la revendication 1, **caractérisée en ce que** ledit trou transversal (19) forme un joint hydraulique en T, conjointement avec la tige creuse (17) de l'aiguille (13) qui se termine dans le trou (19).

6. Seringue (10) selon la revendication 1, **caractérisée en ce que** ladite structure (18) est formée de plastique ou de métal.
